(19) Europäisches Patentamt
European Patent Office
Office européen des brevets

(11) Veröffentlichungsnummer: **0 184 790**
**A2**

# (12) EUROPÄISCHE PATENTANMELDUNG

(21) Anmeldenummer: **85115552.3**

(22) Anmeldetag: **06.12.85**

(51) Int. Cl.⁴: **B 01 J 35/02**

(30) Priorität: **12.12.84 DE 3445289**

(43) Veröffentlichungstag der Anmeldung: **18.06.86**
**Patentblatt 86/25**

(84) Benannte Vertragsstaaten: **BE DE FR GB NL**

(71) Anmelder: **BASF Aktiengesellschaft,**
**Carl-Bosch-Strasse 38, D-6700 Ludwigshafen (DE)**

(72) Erfinder: **Hofmann, Friedbert, Dr., Rehbachstrasse 34a, D-6708 Neuhofen (DE)**
Erfinder: **Krabetz, Richard, Dr., Unterer Waldweg 8, D-6719 Kirchheim (DE)**
Erfinder: **Schwarzmann, Matthias, Dr., 54 Carl-Bosch-Strasse, D-6703 Limburgerhof (DE)**

## (54) Geformter Katalysator für heterogen katalysierte Reaktionen.

(57) Die Anmeldung betrifft einen geformten Katalysator für heterogenkatalysierte Reaktionen, bestehend aus einem zu Hohlzylindern geformten katalytisch aktiven Material oder aus einem zu Hohlzylindern geformten inerten Trägermaterial, auf das eine katalytisch aktive Masse aufgebracht ist, wobei der Aussendurchmesser der Hohlzylinder 3 bis 20 mm, der Innendurchmesser das 0,1 bis 0,7-fache des Aussendurchmessers und die Länge das 0,2- bis 2-fache des Aussendurchmessers beträgt. Die Stirnfläche des Hohlzylinders ist so gekrümmt, dass der Radius der Krümmung das 0,4- bis 5-fache des Aussendurchmessers beträgt.

Die neuen Katalysatoren sind insbesondere für partielle Oxidationen in der Gasphase geeignet.

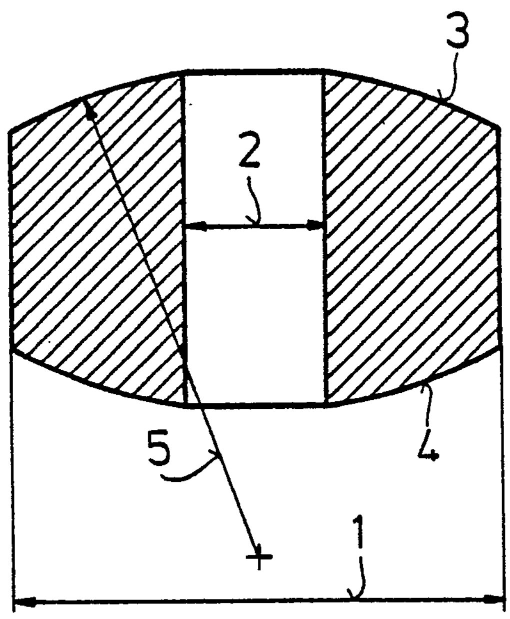


EP 0 184 790 A2

Geformter Katalysator für heterogen katalysierte Reaktionen

Die Anmeldung betrifft geformte Katalysatoren für heterogenkatalysierte Reaktionen, wie sie z.B. fest angeordnet, als Schüttungen, bei Gasphasen- oder Flüssigphasenreaktionen eingesetzt werden.

Die Aufgabe bestand darin, Katalysatoren zu entwickeln, die vorzugsweise geeignet sind für exotherme oder endotherme Reaktionen, die zu mehr als einem Produkt führen, so daß die selektive Umsetzung der Rohstoffe zu einem bestimmten Produkt von entscheidender Bedeutung ist. Derartige, insbesondere Gasphasenreaktionen müssen aus Gründen einer optimalen Temperaturführung in Reaktoren mit einer hohen inneren Wärmetauschfläche, beispielsweise sogenannte Rohrbündelreaktoren, durchgeführt werden, um durch rasche Abführung oder Zufuhr der Reaktionswärme die Ausbildung größerer lokaler Temperaturunterschiede in der Katalysatorschüttung und damit die Bildung von unerwünschten Nebenprodukten zu verhindern.

Für in Schüttungen fest angeordnete Katalysatoren sind vielfältige Formen bekannt, beispielsweise Kugeln, Voll- und Hohlzylinder, Sterne, Speichenradformen und Waben. Die in der Praxis am meisten gebräuchlichen Katalysatorformen sind Kugeln und Voll- bzw. Hohlzylinder. Diese Formen können durch relativ leicht beherrschbare Techniken, wie Aufbaugranulierung (Kugeln), Tablettieren, Extrudieren bzw. Strangpressen zu Voll- bzw. Hohlzylinder, mit im allgemeinen technisch ausreichender mechanischer Festigkeit hergestellt werden. Es ist weiterhin bekannt, daß die Form und die Abmessungen der Katalysatorteilchen direkt oder indirekt den Druckverlust der Katalysatorschüttung und damit die Energiekosten, die Selektivität bzw. den spezifischen Rohstoffverbrauch, die in starkem Maße von den Stoff- und Wärmetransportwiderständen in dem Porengefüge der Katalysatorformlinge bzw. der Länge der Diffusionswege abhängig sind, die Volumenaktivität bzw. die Raumzeitausbeute, die von der Menge aktiver Katalysatormasse je Einheit des Schüttvolumens mitbestimmt wird und die mechanische Festigkeit der Formlinge und damit die Lebensdauer beeinflussen.

Nicht beachtet wurde bisher der Einfluß der Katalysatorform auf die Verweilzeitverteilung in technischen Reaktoren, die sich sowohl auf die Reaktorleistung als auch auf den Umfang der Nebenreaktionen auswirkt. Die Streuung der Verweilzeit ist u.a. davon abhängig, wie einheitlich hinsichtlich des Lückenvolumens und damit des Druckverlustes über den Reaktorquerschnitt beispielsweise die Rohre eines Rohrbündelreaktors innerhalb der Stillstandszeit einer technischen Anlage gefüllt werden können. Die Stillstandszeit ist im Hinblick auf den Produktionsausfall in der Regel so eng bemessen, daß auf einen zeitaufwendigen Druckverlustabgleich

Gr/Kl

der Reaktionsrohre verzichtet werden muß. In Rohrbündelreaktoren, bei denen das wärmeführende Medium durch die Rohre geleitet wird, während der Katalysator in die Zwischenräume gefüllt wird, ist ein Druckverlustabgleich über den Reaktorquerschnitt praktisch nicht möglich.

Es ist weiter bekannt, daß ein einheitlicher Druckverlust über den Reaktorquerschnitt umso schwerer zu erreichen ist, je weniger abriebfest ein Katalysator ist.

Die in der Praxis bis heute verwendeten Katalysatorformen haben hinsichtlich der oben genannten Gesichtspunkte verschiedene Nachteile. So weisen Kugeln zwar relativ zu Zylindern im allgemeinen eine bessere Abriebfestigkeit auf, nachteilig ist aber der größere Druckverlust und der längere Diffusionsweg und damit die schlechtere Selektivität von Kugeln und Vollzylindern im Vergleich zu Hohlzylindern von gleichem Außendurchmesser.

Es wurde nun gefunden, daß ein neuer geformter Katalysator für heterogenkatalysierte Reaktionen, bestehend aus einem zu Hohlzylindern geformten katalytisch aktiven Material oder aus einem zu Hohlzylindern geformten inerten Trägermaterial, auf das eine katalytisch aktive Masse aufgebracht ist, wobei der Außendurchmesser der Hohlzylinder 3 bis 20 mm, der Innendurchmesser das 0,1- bis 0,7-fache des Außendurchmessers und die Länge das 0,2- bis 2-fache des Außendurchmessers beträgt, die oben beschriebenen Nachteile vermeidet und die geschilderte Aufgabe erfüllt, wenn die Stirnflächen des Hohlzylinders gekrümmt sind, wobei der Radius der Krümmung das 0,4- bis 5-fache des Außendurchmessers beträgt.

Aus fertigungstechnischen Gründen kann der Durchmesser der auf die Stirnflächen des zylindrischen Teiles aufgesetzten Kuppen bis etwa 0,2 mm kleiner sein als der Außendurchmesser des zylindrischen Teiles.

Die Figur 1 veranschaulicht einen möglichen Querschnitt der neuen Katalysatoren, worin 1 den Außendurchmesser, 2 den Innendurchmesser, 3 und 4 die Stirnflächen des Hohlzylinders und 5 den Krümmungsradius der Stirnflächen bedeuten. Die Figur 2 zeigt zwei Grenzfälle a und b für die erfindungsgemäßen Katalysatoren, worin für a der Krümmungsradius (5) der Stirnflächen etwa das 0,5fache des Außendurchmessers (1) und der Innendurchmesser (2) das 0,17fache des Außendurchmessers (1) und für b der Krümmungsradius der Stirnflächen etwa das 2,0fache des Außendurchmessers und der Innendurchmesser etwa das 0,7fache des Außendurchmessers bedeuten.

Die neue Katalysatorform vereinigt die Vorzüge von Hohlzylindern hinsichtlich der Selektivität mit einer im Vergleich zu Voll- und Hohlzylindern besseren Abriebfestigkeit und einem geringen Strömungswiderstand (Druckverlust). Insbesondere überraschend ist die im Vergleich zu den gebräuchlichen Hohlzylindern mit ebenen Stirnflächen geringere Streuung der Schüttdichten bzw. des Druckverlustes beim Füllen technischer Rohre. Die enge Streubreite ergibt in technischen Reaktoren eine bessere durchschnittliche Selektivität und/oder Raumzeitausbeute.

Die erfindungsgemäßen Katalysatoren werden nach an sich bekannten Verfahren hergestellt, beispielsweise durch Fällung der Oxide, Oxidhydrate, Hydroxide oder anderer schwerlöslicher Verbindungen der Komponenten aus Lösungen ihre Salze, oder durch intensives Vermischen der oxidischen und/oder salzförmigen Ausgangsstoffe, die gegebenenfalls in Wasser oder organischen Lösungsmitteln gelöst oder/und suspendiert sind, anschließende Trocknung und gegebenenfalls thermische Zersetzung der Salze, Vermahlen zu einer geeigneten tablettierfähigen Teilchengröße, Verformung zu der erfindungsgemäßen Form in geeigneten Tablettiermaschinen und gegebenenfalls anschließende thermische Behandlung bei erhöhten Temperaturen in oxidierender, reduzierender oder inerter Atmosphäre. Der Katalysatormasse können in irgendeiner Herstellungsstufe von der Verformung Formhilfsmittel wie Graphit, Ruß, Stearinsäure, Stärke, Polyacrylsäure, Mineralöl, pflanzliches Öl, Methylcellulose u.a., sowie Verstärkungsmittel wie anorganische Fasern, beispielsweise Glasfasern, Asbestfasern und dgl., oder anorganische Pulver beispielsweise Metallpulver, Metallflocken, sogenannte inerte Trägerstoffe, beispielsweise $SiO_2$, Metallsilikate und Aluminiumsilikate, Aluminiumoxide, -hydroxide, -oxidhydrate, MgO, $TiO_2$, $ZrO_2$, $Nb_2O_3$, Bimsstein, Siliziumcarbid und Magnesiumsilikate zugegeben werden.

Die neue Katalysatorform ist auch vorteilhaft bei sogenannten Trägerkatalysatoren, die in der Weise hergestellt werden, daß die aktiven Komponenten durch Tränken, Eindampfen, Aufsprühen einer Lösung oder Suspension der Komponenten in der oxidischen oder Salzform auf den vorgeformten inerten Träger aufgebracht werden. Während der Herstellung derartiger, insbesonders ringförmiger Katalysatoren agglomeriert in der Regel ein größerer oder kleinerer Anteil der Einzelteilchen häufig über die Stirnflächen zu sogenannten Zwillingen oder Drillingen, die oft auch nach einer aufwendigen Siebung eines Produktionsansatzes nicht völlig abgetrennt werden können und daher die Dichteschwankung beim Befüllen eines Reaktors verstärken. Bei der neuen Katalysator- bzw. Trägerform ist der Umfang der Agglomeration vernachlässigbar gering.

Hinsichtlich der chemischen Zusammensetzung der Katalysatoren bestehen keine Einschränkungen.

Die neue Katalysatorform ist vorzugsweise geeignet für partielle Oxidationsreaktionen in der Gasphase, insbesondere die selektive Umsetzung von Propylen zu Acrolein/Acrylsäure, von Isobuten oder tert.-Butylalkohol zu Methacrolein/Methacrylsäure, (Meth)acrolein zu (Meth)acrylsäure, die oxidative Dehydrierung von Isobuttersäure(ester) zu Methacrylsäure(ester), die Oxidation von n-Butan, n-Buten und Benzol zu Maleinsäure, von o-Xylol zu Phthalsäureanhydrid, von Toluol und substituierten Toluolen zu substituierten Benzaldehyden und Benzoesäuren. Obwohl hinsichtlich der Katalysatorzusammensetzung keine prinzipiellen Einschränkungen existieren, so ergeben sich besondere Vorteile insbesondere bei Katalysatoren, deren Zusammensetzung der allgemeinen Formel:

$$Mo_a V_b W_c A_d B_e C_f D_g O_x$$

entspricht, wobei A = ein oder mehrere Elemente aus der Gruppe Ni, Co, Fe, Bi, Cu, Rh, Ru, Re,Pd, Cr, Mn, Sn, Ce, Ag, Pb, Sb, B = ein oder mehrere Elemente aus der Gruppe P, B, As, C = ein oder mehrere Elemente aus der Gruppe der Alkali- und/oder Erdalkalimetalle, sowie Tl, und D = ein oder mehrere Elemente aus der Gruppe der Seltenen Erden, Nb, Ta, U

a = 0 - 12; b = 0 - 12; c = 0 - 12; a + b + c = 12, d = 0 - 12;
e = 0 - 3; f = 0 - 3; g = 0 - 12

bedeuten, und die ggf. auf inerte Träger aufgebracht oder mit inerten Trägermaterialien vermischt sein können.

Aktive Massen dieser Zusammensetzung lassen sich im allgemeinen nur unter Verlust an Selektivität zu ausreichend druck- und abriebfesten Kugeln, Voll- und Hohlzylindern verformen. Diese Nachteile sind bei den erfindungsgemäßen Katalysatoren deutlich weniger ausgeprägt.

Die Vorteile der erfindungsgemäßen Katalysatoren gegenüber herkömmlichen Katalysatoren in Form von Hohlzylindern gehen aus folgenden Beispielen hervor.

Beispiel 1

Es wurde eine katalytisch aktive Masse der Zusammensetzung entsprechend der Formel

$$Mo_{10}V_1P_1As_{0,2}Cu_{0,25}W_{0,1}K_{0,12}O_x$$

auf folgendem Wege hergestellt:

300 Gewichtsteile $MoO_3$, 18,9 Gewichtsteile $V_2O_5$, 24 Gewichtsteile $H_3PO_4$ (85 %ig), 5,4 Gewichtsteile $As_2O_5 \cdot H_2O$, 3,3 Gewichtsteile CuO wurden in 3000 Gewichtsteilen destilliertem Wasser unter Rühren 3 Stunden am Rückfluß zum Sieden erhitzt. Nach Zugabe von 1,35 Gewichtsteilen KOH (85 %) und 8 Gewichtsteilen Wolframphosphorsäure wurde die Lösung weitere 3 Stunden zum Sieden erhitzt. Nach Zugabe von 100 Gewichtsteilen Kieselgur wurde die Suspension zur Trockene eingedampft.

Die oxidische Masse wurde anschließend auf eine Körnung kleiner 1,2 mm gemahlen und mit 2 Gew.% Graphitpulver gemischt und dann zu Tabletten folgender Abmessung verpreßt:

A) Ringe mit gleichem Außen- und Innendurchmesser wie unter B) angegeben. Die Stirnflächen der Ringe waren im Unterschied zu B) nicht planparallel, sondern entsprechend einem Krümmungsradius von 7 mm gewölbt. Die Achsenlänge betrug 5 mm (erfindungsgemäßer Katalysator A).

B) Ringe mit einem Außendurchmesser von 7 mm, einem Innendurchmesser von 3 mm und einer Länge von 5 mm (Vergleichskatalysator B).

Zur Simulation des Füllvorganges bei einem technischen Rohrbündelreaktor wurden jeweils 0,75 kg Formlinge innerhalb von 30 sec mit gleichmäßiger Geschwindigkeit in ein senkrecht stehendes Glasrohr von 150 cm Länge und 25 mm Innendurchmesser eingefüllt und anschließend die Füllhöhe an einer Skala abgelesen.

Die Tabelle gibt die Anzahl der Füllversuche von jeweils 100 Versuchen an, bei denen die Abweichung der Füllhöhe von der mittleren Füllhöhe weniger als $\pm$ 1 % beträgt.

| Abweichung weniger als | Zahl der Versuche (Rohre) erfindungsgemäßer Katalysator A | Vergleichskatalysator B |
|---|---|---|
| $\pm$ 0,5 % | 84 | 50 |
| $\pm$ 1,0 % | 98 | 74 |

Bei dem erfindungsgemäßen Katalysator ist die Streuung der Füllhöhen und damit des Lückenvolumens und des Druckverlustes wesentlich geringer als bei dem Vergleichskatalysator.

Zur Prüfung der effektiven Abriebfestigkeit wird jeweils eine Katalysatorcharge von 0,75 kg Anfangsgewicht 50 mal in das Rohr eingefüllt und nach Absieben des Staubes der Gewichtsverlust bestimmt. Er beträgt beim Vergleichskatalysator B 2,4 Gew.%, beim erfindungsgemäßen Katalysator A hingegen nur 1,8 Gew.%.

Beispiel 2

Aus einer Steatitmasse, bestehend aus 83 Gew.% Speckstein, 9 % plastischem Ton und 8 % Feldspat, werden nach Zusatz von Wasser und Methylzellulose zwei Formlinge A und B gepreßt und anschließend bei 1000°C geglüht.

Der Formling A ist eine gelochte Kugel entsprechend der Abb. 2a mit einem Außendurchmesser des zylindrischen Teils von 8 mm, einem 1 mm breiten Bund und einem Lochdurchmesser von 4 mm. Die Länge beträgt 6 mm und der Krümmungsradius 4,5 mm (Träger A). Der Formling B ist ein Hohlzylinder von 8 mm Außendurchmesser, 6 mm Länge und 4 mm Innendurchmesser (Träger B, Vergleichskatalysator).

Parallel wird ein katalytisch aktives Material der Zusammensetzung

$$Mo_{12}NiCo_7Fe_3Bi_2B_2Sb_{0,1}K_{0,14}O_{56,7}$$

nach Angaben des Beispiels 1 der GB-A 1 491 750 hergestellt. Das Material wird auf eine Teilchengröße von kleiner 30 µm gemahlen.

Jeweils 157 Gewichtsteile des katalytisch aktiven Pulvers werden in 143 Gewichtsteilen Wasser suspendiert, die Suspensionen werden mit einer Geschwindigkeit von 0,16 kg/kg, h auf je 1000 Gewichtsteile der Trägerformlinge A und B aufgesprüht. Die Träger befinden sich dabei in einer Drehtrommel, die mit einer Drehzahl von 35 Umdrehungen pro Minute rotiert und die indirekt auf 40 bis 50°C beheizt wird. Die Treibgasmenge, mit der die Suspension durch eine Zweistoffzerstäubungsdüse versprüht wird, beträgt 7,5 $Nm^3$/kg, h Luft einer Temperatur von 22°C. Nach Beendigung der Beschichtung werden die erhaltenen Schalenkatalysatoren bei 100°C nachgetrocknet.

Der Anteil agglomerierter Teilchen betrug beim Katalysator A weniger als 1 Gew.%, beim Katalysator B ca. 4 Gew.%.

Die Simulation des Einfüllvorgangs in technische Rohre gemäß Beispiel 1 und Vergleichsbeispiel 1, wobei jeweils 680 ml der Katalysatoren eingesetzt wurden, ergab die in Tabelle 2 zusammengefaßten Ergebnisse:

Tabelle 2: Abweichung der Füllhöhe von der mittleren Füllhöhe

| Abweichung weniger als | Zahl der Versuche erfindungsgemäßer Katalysator A | Vergleichskatalysator B |
|---|---|---|
| ± 1,0 % | 80 | 62 |
| ± 1,5 % | 100 | 72 |
| ± 2,0 % | 100 | 82 |

Patentansprüche

1. Geformter Katalysator für heterogenkatalysierte Reaktionen, bestehend aus einem zu Hohlzylindern geformten katalytisch aktiven Material oder aus einem zu Hohlzylindern geformten inerten Trägermaterial, auf das eine katalytisch aktive Masse aufgebracht ist, wobei der Außendurchmesser der Hohlzylinder 3 bis 20 mm, der Innendurchmesser das 0,1 bis 0,7-fache des Außendurchmessers und die Länge das 0,2- bis 2-fache des Außendurchmessers beträgt, dadurch gekennzeichnet, daß die Stirnflächen des Hohlzylinders gekrümmt sind, wobei der Radius der Krümmung das 0,4- bis 5-fache des Außendurchmessers beträgt.

2. Katalysator nach Anspruch 1, dadurch gekennzeichnet, daß der Radius der Krümmung das 0,6- bis 2,0-fache des Außendurchmessers beträgt.

3. Verwendung der Katalysatoren nach Anspruch 1 und 2 für die partielle Oxidation in der Gasphase.

Zeichn.

FIG.1

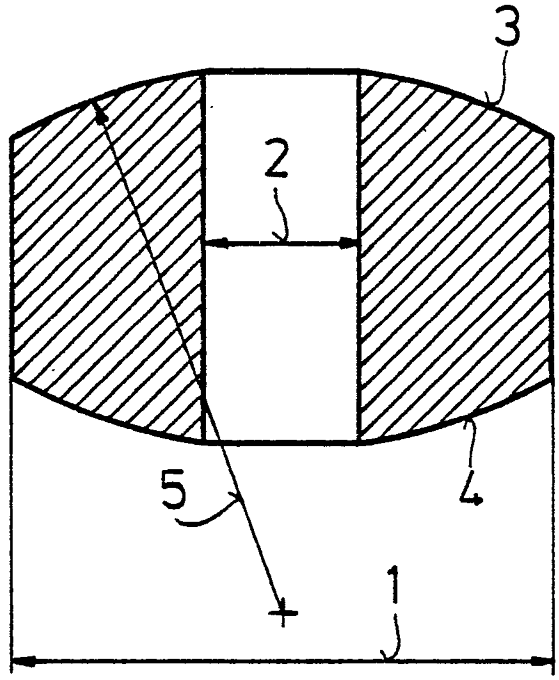


FIG.2

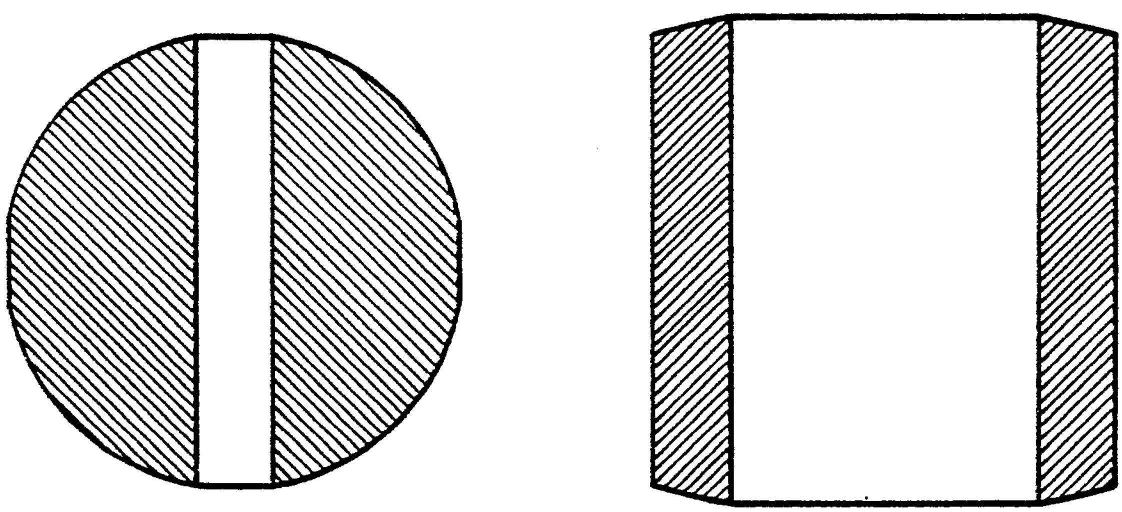

a b